# EUROPEAN PATENT SPECIFICATION

(11) **EP 3 727 553 B1**
(45) Date of publication and mention of the grant of the patent: **24.11.2021**
(21) Application number: 18709115.2
(22) Date of filing: 03.01.2018
(51) Int. Cl.: A61M 25/10

(54) **METHODS OF MANUFACTURING A BALLOON WITH INTEGRAL SCORING ELEMENT**
VERFAHREN ZUR HERSTELLUNG EINES BALLONS MIT INTEGRALEM RITZELEMENT
MÉTHODES DE FABRICATION D´UN BALLONNET AVEC ÉLÉMENT D'ENTAILLE INTÉGRÉ

(43) Date of publication of application: 28.10.2020
(60) Divisional application: 21201867.5
(73) Proprietor: C.R. Bard, Inc., Tempe, AZ 85281 (US)
(72) Inventor: RONAN, Allan, Wexford (IE); GILES, Ciaran, Wexford (IE)
(74) Representative: Hoffmann Eitle
(86) International application number: PCT/US2018/012196
(87) International publication number: WO 2019/135741

(56) References cited:
- EP-A1- 2 564 890
- EP-A1- 2 644 171
- WO-A1-2012/044477
- US-A1- 2011 264 185
- US-A1- 2012 130 407

## Description

### TECHNICAL FIELD

This disclosure pertains to manufacturing methods of devices for providing endovascular treatment with a scorign balloon.

### BACKGROUND

Balloon dilatation catheters are used to treat lesions in vessels. However, difficulties are encountered in navigating tortuous anatomy and safely crossing very tight lesions. Moreover, some lesions are difficult to dilate using just a balloon, and require a focused force to dilate the lesion at safe inflation pressures.

U.S. Pat. No. 6,394,995 to Solar et al. describes a system used to provide enhanced force to treat a lesion using a scoring wire attached to the balloon. Typically, the scoring wire extends through a separate lumen in the catheter shaft. Aside from being complicated to manufacture, the scoring wire may in some circumstances move circumferentially along the balloon, which may make it more challenging to provide the desired focused force.

To alleviate this issue, others have proposed a balloon including integral scoring elements in the form of surface protuberances (e.g., U.S. Patent Application Publication No. 2009/0234283). In this known example, the protuberances are formed during extrusion of a parison, which must then be blown into a balloon, requiring a complicated two-step process.

US 2012/130407 A1 discloses a scoring balloon including a generally cylindrical and straight body portion and both ends of the body portion and cone portions. Tapering sections taper from a height generally consistent with the central section to a lower height along the direction of taper of the balloon end cones.

Additionally, with either the integral scoring element or the separate scoring wire, the scoring force provided is linear according to the position of the wire or protuberance in alignment with a longitudinal axis of the balloon. This can serve as a limitation on the effectiveness of the treatment provided, depending on the needs of the clinician in terms of a particular desired outcome.

Accordingly, it would be desirable to be able to form an integral scoring element during the process of blow molding a parison into a balloon, which avoids the issues with forming the scoring element during extrusion or later mechanical attachment.

### Summary

An object of the invention is to provide a method of manufacturing a medical balloon in terms of claim 1.

According to a first aspect of the disclosure, a method of manufacturing a medical balloon is provided. The method comprises forming the medical balloon with a scoring element projecting from an outer surface of the balloon along at least a barrel section by expanding a tubular parison in a mold cavity including a groove corresponding in shape to the scoring element.

The forming step may include forming the scoring element along the barrel section and at least one end section of the balloon. The forming step may alternatively comprise forming the scoring element as a single scoring element, such as a single longitudinally extending scoring element. The forming step may include forming a plurality of scoring elements. The forming step may further include forming the scoring element as a helically extending scoring element, a circumferentially extending scoring element, or any combination of the foregoing.

The method further includes the step of forming the groove in a mold cavity. The step of forming the medical balloon may also comprise applying fluid under pressure to an interior of the parison. The forming step may also comprise heating a mold section associated with the mold cavity.

A further aspect of the disclosure pertains to a medical balloon, which does not form part of the invention. The medical balloon comprises a body including a working surface having an integral scoring element projecting therefrom. The integral scoring element may extending circumferentially around the body. The working surface may include a plurality of integral scoring elements elongated in a circumferential direction.

Still a further aspect of the disclosure pertains to a medical balloon not forming part of the invention and comprising a body including a working surface having an integral scoring element projecting therefrom, the integral scoring element extending helically around the body. The working surface may include a plurality of integral scoring elements extending around the balloon in a helical direction.

This disclosure additionally pertains to a mold for forming a medical balloon having with tapered end sections, a barrel section, and at least one scoring element. The mold, which does not form part of the invention, comprises a plurality of mold sections forming a mold cavity corresponding in shape to the medical balloon, at least one of the mold sections including a groove within the cavity corresponding to the scoring element. The groove may extend longitudinally along the cavity, circumferentially around the cavity, or helically along the cavity.

### BRIEF DESCRIPTION OF THE DRAWING FIGURES

The above and further advantages of the present invention may be better understood by referring to the following description in conjunction with the accompanying drawings in which:
Figure 1 is a perspective view of a partial catheter with a balloon;
Figure 2 is a cross-sectional view taken along line 2-2 of Figure 1;
Figure 3 is a top perspective view of a balloon catheter;
Figure 4 is a side view of a balloon catheter;
Figure 5 is a perspective view of a tubular parison for forming a medical balloon;
Figure 6 is a side, cross-sectional view of a mold for forming the medical balloon;
Figure 7 is a perspective view of the medical balloon formed according to Figure 6;
Figure 8 is a side, cross-sectional view of a mold for forming a medical balloon with a plurality of longitudinally extending scoring elements along a working surface associated with a barrel section of the balloon;
Figures 8a and 8b are cross-sectional views of one mold section for forming a balloon with an integral scoring element;
Figure 9 is a perspective view of the medical balloon formed according to Figure 8;
Figures 9a and 9b are partially cutaway, partially cross-sectional views of a balloon with different shapes of integral scoring elements;
Figure 10 is a side, cross-sectional view of a mold for forming the medical balloon with a plurality of longitudinally extending scoring elements along a working surface associated with a barrel section of the balloon, as well as along tapered end sections of the balloon;
Figure 11 is a perspective view of the medical balloon formed according to Figure 10;
Figure 12 is a plan view of a mold section for forming the medical balloon with a plurality of helically extending scoring elements along a working surface associated with a barrel section of the balloon;
Figure 13 is a side view of the medical balloon formed according to Figure 12;
Figure 14 is a plan view of a mold section for forming the medical balloon with a plurality of circumferentially extending scoring elements along a working surface associated with a barrel section of the balloon; and
Figure 15 is a side view of the medical balloon formed according to Figure 14.

The drawings are not necessarily drawn proportionally or to scale. For example, the dimensions of some of the elements may be exaggerated relative to other elements for clarity or several physical components may be included in one functional block or element. Further, sometimes reference numerals may be repeated among the drawings to indicate corresponding or analogous elements.

### DETAILED DESCRIPTION

In the following detailed description, numerous specific details are set forth to provide a thorough understanding of the disclosed concepts. Those of ordinary skill in the art will know that the disclosed inventions may be practiced without these specific details. In other instances, well-known methods, procedures, components, or structures may not have been described in detail so as not to obscure the disclosed inventions.

The description provided below and in regard to the figures applies to all embodiments unless noted otherwise, and features common to each embodiment are similarly shown and numbered.

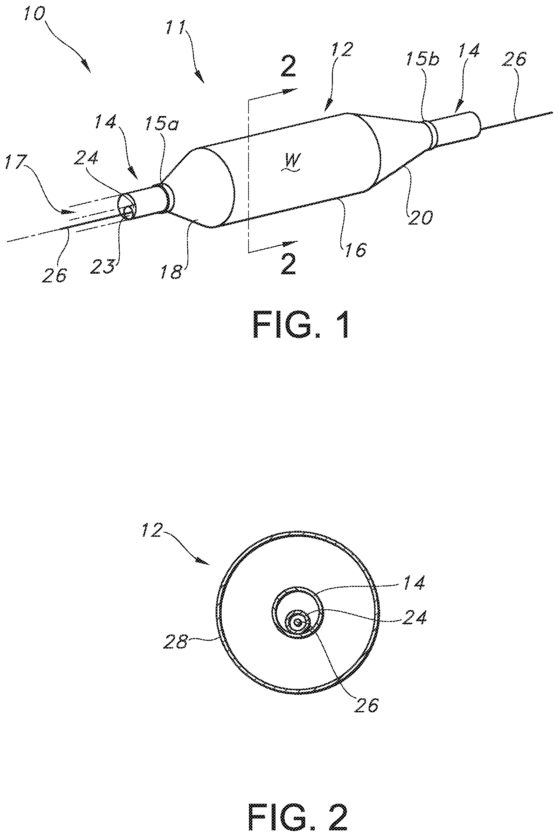

Referring first to Figures 1, 2, 3, and 4, a typical endovascular catheter 10 is shown, which includes a distal portion 11 with a balloon 12 mounted on a catheter tube 14, such as an elongated tubular body having at least one open end for receiving a fluid for use in causing the balloon to selectively inflate. The balloon 12 has a body comprising an intermediate section 16, or "barrel" having the working surface W, and end sections 18, 20. In one possible embodiment, the end sections 18, 20 reduce in diameter or taper to join the intermediate section 16 to the catheter tube 14 (and thus sections 18, 20 are generally termed cones or cone sections). The balloon 12 is sealed at balloon ends (proximal 15a and distal 15b) on the end sections 18, 20 to allow the inflation of the balloon 12 via one or more inflation lumens 17 extending within catheter tube 14 and communicating with the interior of the balloon 12.

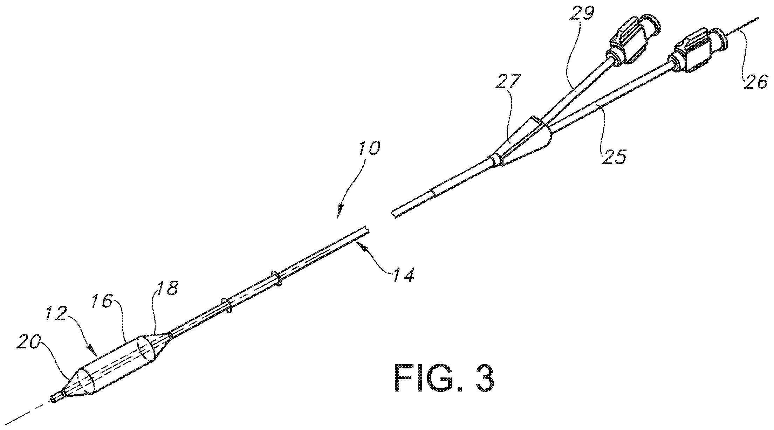

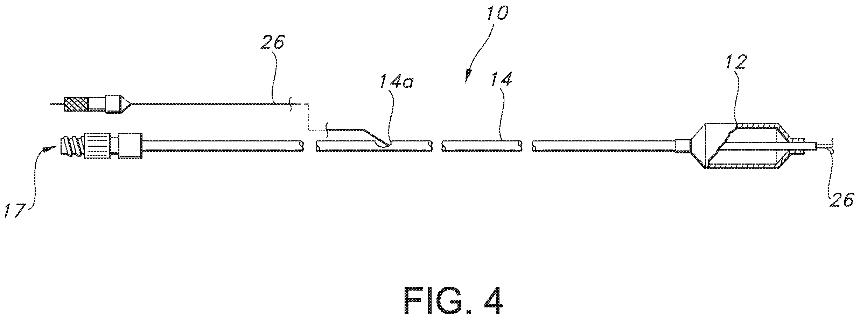

The catheter tube 14 also includes an elongated, tubular shaft 24 forming a guidewire lumen 23 that directs the guidewire 26 through the catheter 10. As illustrated in Figure 3, this guidewire 26 may be inserted through a first port 25 of a connector, such as a hub 27, into the lumen 23 to achieve an "over the wire" (OTW) arrangement, but could also be provided in a "rapid exchange" configuration in which the guidewire 26 enters the lumen through a lateral opening 14a closer to the distal end (see Figure 4). A second port 29 may also be associated with catheter 10, such as by way of connector (e.g., hub 27), for introducing a fluid (e.g., saline, a contrast agent, or both) into the interior of the balloon 12 via the inflation lumen 17.

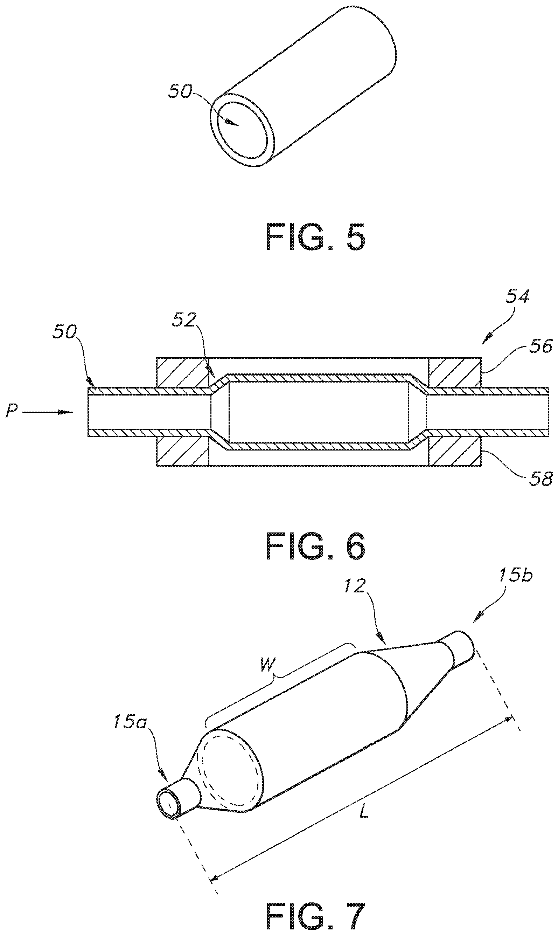

From Figures 5, 6 and 7, it can be understood that the balloon 12 is formed as a separate component during a blow molding process, in which a parison typically in the form of a tubular structure or tube 50. Thus tube 50 has a constant outer diameter and thus lacks any surface projections in the illustrated embodiment. The tube 50 may be formed of an expandable polymer material is expanded in a mold cavity 52 of a mold 54 comprised of separable sections 56, 58 under heat and pressure (which may be supplied via a fluid passed into the tube 50 as indicated by arrow P) to form the balloon having the above-described shape. As can be appreciated by comparing Figures 6 and 7, the shape of the mold cavity 52 thus dictates the shape and outer surface characteristics of the resulting balloon 12, including the length of the working surface W relative to the overall length L of the balloon 12 (which is can be appreciated may be adjusted by trimming the ends 15a, 15b).

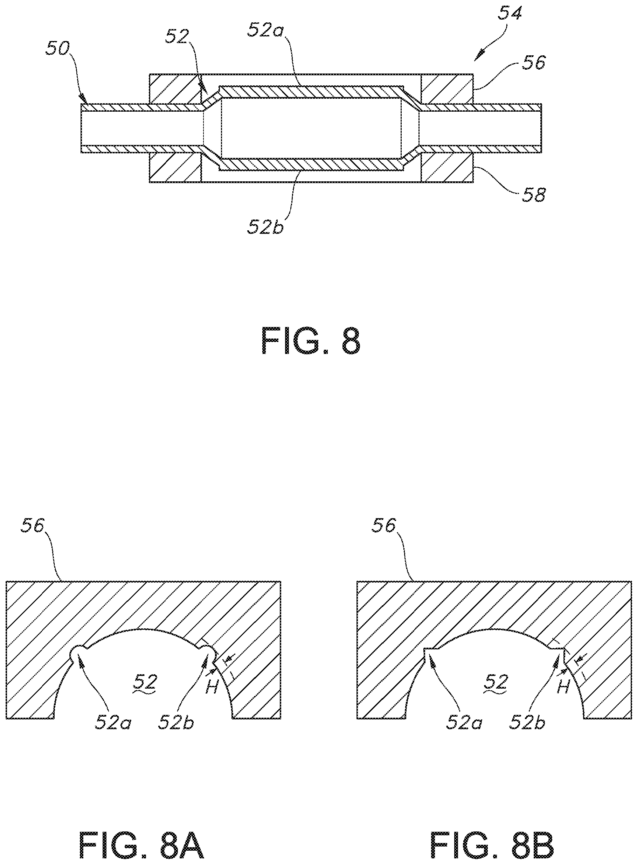

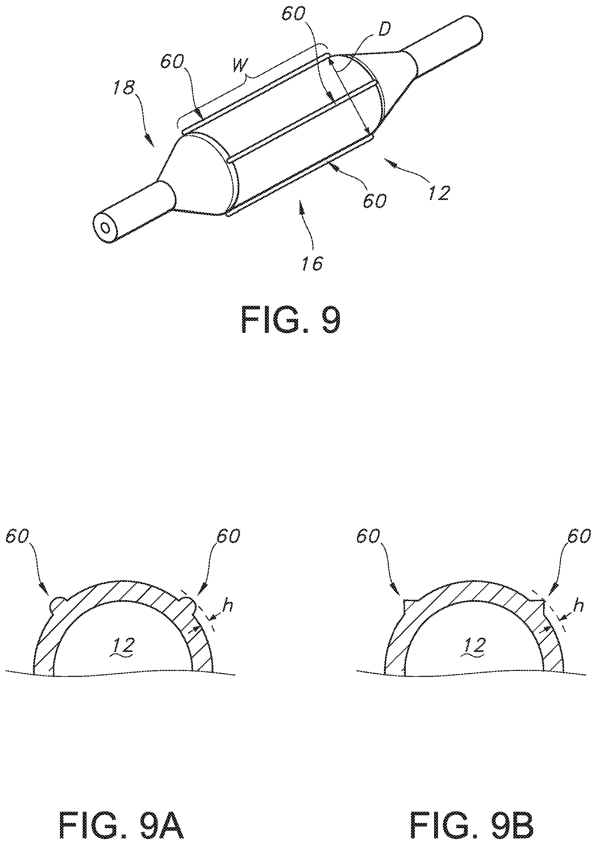

According to one aspect of the disclosure, and with further reference to Figures 8 and 9, the balloon 12 is provided with one or more integral scoring elements 60 (three shown for purposes of illustration, but any number greater than one may be provided). A "scoring element" is generally a structure adapted for cutting something, such as a lesion in a vessel, may take the form of narrow, peripheral ridges and thus project from the working surface W and thus indicate a partial increase in the nominal diameter D of the balloon. This is achieved by providing one or more of the mold sections 56, 58 with a groove 52a, 52b (two shown, but one or three or more may be provided) into which a portion of the tube 50 may expand as a result of the blow molding process (but the remaining portion of the tube that does not extend into the groove of course forms the outer surface of the balloon from which the scoring element projects to provide a treatment effect on expansion of the balloon). The groove or grooves 52a, 52b may be in the form of elongated, narrow, linear grooves formed into the material of the mold sections 56, 58, which may themselves be formed by molding or material removal form the corresponding section once formed.

As can be understood from Figure 9, this technique provides the scoring elements 60 with a corresponding shape in the working surface W of the balloon 12, and also corresponding in number and location to the number of grooves provided in the mold cavity 52. The balloon 12 once formed may of course be removed from the mold 54 and secured to a shaft, such as tube 14, for use as the catheter 10 of any of Figures 1-4.

As can be understood from Figures 8a and 8b, which illustrate one mold half or section 56 (the other of which may be identical, but inverted), the outward end of each groove 52a, 52b is closed off in the radial direction to provide an abutment surface for engaging the parison material when expanded in the mold cavity 52, which thereby defines the shape of the scoring element 60 formed in the wall 28 of the balloon 12. This end may be contoured, and may have a variety of shapes depending on the desired shape of the integral scoring element(s) 60. For example, as shown in Figure 8a, each groove 52a, 52b may have a curved contour for forming a scoring element 60 with a rounded profile, as shown in Figure 9a. Alternatively, as shown in Figure 9a, the closed end of each groove 52a, 52b may have an inverted V-shaped contour for forming a scoring element with a V-shaped profile, as shown in Figure 9b.

The relative height H of each groove 52a, 52b (that is, the distance from the open end to the closed end) may also be varied depending on the desired height of the scoring element(s) 60. As examples, the height may range from about 1 to about 10 thousands of an inch (0.0254-0.254mm). Given the thickness of the material, this would result in a slightly smaller height h of the scoring element(s) 60 formed, but with a similar range of dimensions save for tolerances. In relative terms, the height h of each scoring element 60 may be approximately 2% of the total thickness of the wall 28 of the balloon 12 (that is, if the height of the scoring element is 0.0254 mm, the balloon wall thickness would be approximately 1.27 mm).

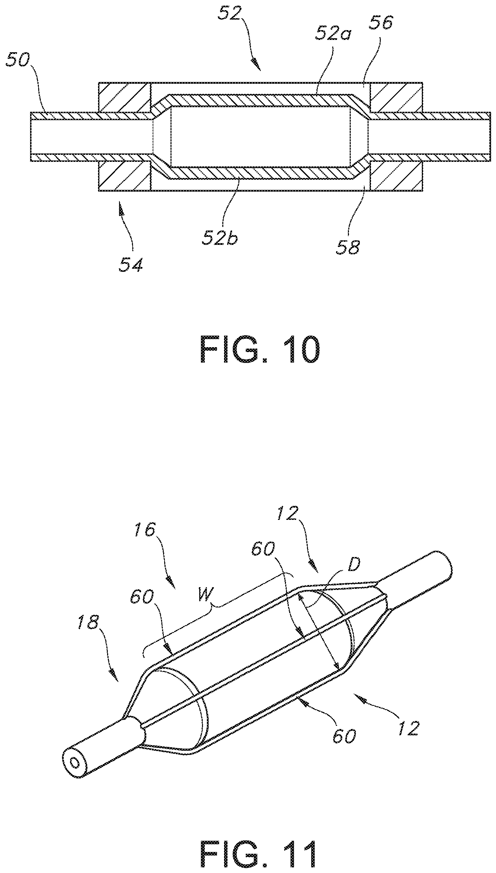

While Figure 9 shows the scoring elements 60 as extending only along the working surface W (and the full extent of it), it can be appreciated that other arrangements are possible. For example, as shown in Figures 10 and 11, the grooves 52a, 52b may be provided along the barrel section 16 of the balloon 12, as well as the tapered or conical end sections 18, 20. This is achieved by simply providing the grooves 52a, 52b along corresponding portions of the mold cavity 52. Likewise, the scoring elements could extend along one part of the working surface W, but not the entirety of it, by simply forming the grooves 52a, 52b according to the desired positioning.

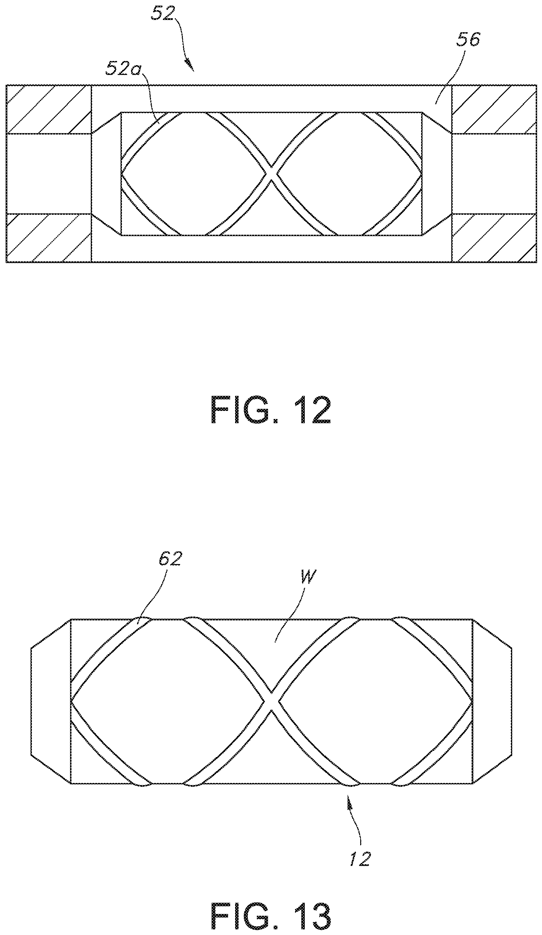

The above embodiments of scoring balloons 12 incorporate linear scoring elements 60, but as can be appreciated, more complex shapes may also be provided. For instance, as shown in Figures 12 and 13, the mold section 56 may be provided with grooves 52a that form a non-linear scoring element 62 on the working surface W of the balloon 12. The grooves 52a may form a single or double helix, as shown, and may be provided in multiple mold sections 56, 58, or just one, depending on the desired modification to the balloon surface. Furthermore, while a generally symmetrical arrangement is shown, different patterns may be provided in different portions of the mold cavity 52.

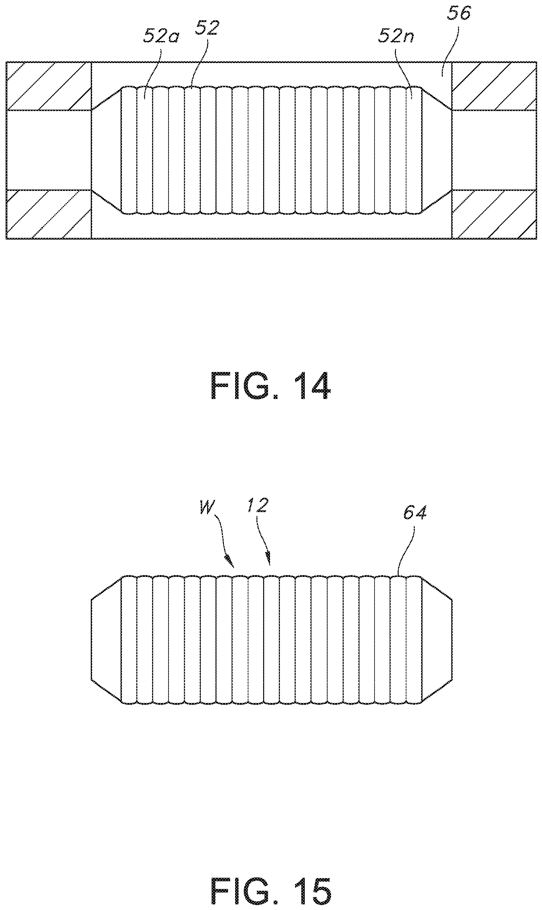

Figures 14 and 15 illustrate yet another example, in which the grooves 52a ... 52n extend in a generally circumferential direction around the mold cavity 52 formed by the mold section 56 (with the other mold section (not shown) being identical or different in construction). The resulting balloon 12 has a plurality of circumferentially arranged scoring elements 64. The particular number provided will of course depend on the number of grooves 52a provided in the mold cavity 52. The grooves 52a ...52n may be juxtaposed, or may be spaced apart, depending on the desired shape of the balloon 12.

Moreover, it is possible to provide grooves 52a ... 52n that get progressively larger (deeper or wider) in the longitudinal direction, which can create a balloon with a more pronounced scoring effect in a portion (e.g., the center) than in other portions (the ends), which may thus allow for a more targeted and effective treatment regimen. Likewise, the situation may be reversed, such that the grooves 52a ... 52n get smaller (in depth or width) from the ends toward the center. It is also possible to provide the circumferential grooves extending over all or any portion of the balloon (i.e., only on the working surface).

As can be appreciated, using the above techniques, it may be possible to provide a single balloon having a plurality of different shapes and sizes of scoring elements along different portions or sections of the balloon, and extending in different directions. Such is not considered possible using mechanical wires or extruded parisons typical of known approaches to providing scoring elements on medical balloons.

The balloon 12 formed using the disclosed method may include a single or multi-layered balloon wall 28 (such as by using a multi-layered tube as the parison), which may be formed of a polymer, such as a polyamide (Nylon, in particular). The balloon 12 may be a non-compliant balloon having a balloon wall 28 that maintains its size and shape in one or more directions when the balloon is inflated. The balloon 12 in such case also has a pre-determined surface area that remains constant during and after inflation, also has a pre-determined length and pre-determined circumference that each, or together, remain constant during and after inflation. However, the balloon 12 could be semi-compliant or compliant instead, depending on the particular use. The catheter 10 may also be used in connection with a variety of treatments, including stents, stent grafts, scoring balloons, and others, without limitation.

The mold 54 may be formed of a metal material. The material may have good heat transfer properties and be readily amenable to milling to form the one or more grooves or grooves 52a in the cavity. Brass is an example of a material from which the mold 54 may be fabricated with anticipated good results in terms of the balloon formation achieved as a result.

Each of the following terms written in singular grammatical form: "a", "an", and the", as used herein, means "at least one", or "one or more". Use of the phrase One or more" herein does not alter this intended meaning of "a", "an", or "the". Accordingly, the terms "a", "an", and "the", as used herein, may also refer to, and encompass, a plurality of the stated entity or object, unless otherwise specifically defined or stated herein, or the context clearly dictates otherwise. For example, the phrases: "a unit", "a device", "an assembly", "a mechanism", "a component, "an element", and "a step or procedure", as used herein, may also refer to, and encompass, a plurality of units, a plurality of devices, a plurality of assemblies, a plurality of mechanisms, a plurality of components, a plurality of elements, and, a plurality of steps or procedures, respectively.

Each of the following terms: "includes", "including", "has", "having", "comprises", and "comprising", and, their linguistic / grammatical variants, derivatives, or/and conjugates, as used herein, means "including, but not limited to", and is to be taken as specifying the stated components), feature(s), characteristic^), parameters), integers), or step(s), and does not preclude addition of one or more additional components), feature(s), characteristics), parameters), integer(s), step(s), or groups thereof. Each of these terms is considered equivalent in meaning to the phrase "consisting essentially of. Each of the phrases "consisting of and "consists of, as used herein, means "including and limited to". The phrase "consisting essentially of' means that the stated entity or item (system, system unit, system sub-unit device, assembly, sub-assembly, mechanism, structure, component element or, peripheral equipment utility, accessory, or material, method or process, step or procedure, sub-step or sub-procedure), which is an entirety or part of an exemplary embodiment of the disclosed invention, or/and which is used for implementing an exemplary embodiment of the disclosed invention, may include at least one additional feature or characteristic" being a system unit system sub-unit device, assembly, sub-assembly, mechanism, structure, component or element or, peripheral equipment utility, accessory, or material, step or procedure, sub-step or sub-procedure), but only if each such additional feature or characteristic" does not materially alter the basic novel and inventive characteristics or special technical features, of the claimed item.

The term "method", as used herein, refers to steps, procedures, manners, means, or/and techniques, for accomplishing a given task including, but not limited to, those steps, procedures, manners, means, or/and techniques, either known to, or readily developed from known steps, procedures, manners, means, or/and techniques, by practitioners in the relevant field(s) of the disclosed invention.

Terms of approximation, such as the terms about, substantially, approximately, etc., as used herein, refers to ± 10 % of the stated numerical value.

It is to be fully understood that certain aspects, characteristics, and features, of the invention, which are, for clarity, illustratively described and presented in the context or format of a plurality of separate embodiments, may also be illustratively described and presented in any suitable combination or sub-combination in the context or format of a single embodiment. Conversely, various aspects, characteristics, and features, of the invention which are illustratively described and presented in combination or sub-combination in the context or format of a single embodiment may also be illustratively described and presented in the context or format of a plurality of separate embodiments.

## Claims

1. A method of manufacturing a medical balloon (12), comprising:
forming the medical balloon (12) with an elongated scoring element (60) projecting from an outer surface of the balloon and extending in a longitudinal direction along at least a barrel section (16) by expanding a tubular parison (50) in a mold cavity (52) including a groove (52a, 52b) corresponding in shape to the scoring element (60), the tubular parison (50) having a constant outer diameter and lacking any surface projections.

2. The method of claim 1, wherein the forming step includes forming the scoring element (60) along the barrel section (16) and at least one end section (18, 20) of the medical balloon (12).

3. The method of claim 1 or 2, wherein the forming step includes forming the scoring element (60) as a single scoring element.

4. The method of claim 3, wherein the forming step includes forming the scoring element (60) as a single longitudinally extending scoring element.

5. The method of claim 1 or 2, wherein the forming step includes forming a plurality of scoring elements (60).

6. The method of claim 1, wherein the forming step includes forming the scoring element (60) as a helically extending scoring element.

7. The method of any of the preceding claims, wherein the linear groove (52a, 52b) has an inverted V-shaped contour for forming the scoring element with a V-shaped profile.

8. The method of any of the preceding claims, further including the step of forming the groove (52a, 52b) in a mold cavity.

9. The method of any of the preceding claims, wherein the forming step comprises applying fluid under pressure to an interior of the tubular parison (50).

10. The method of any of the preceding claims, wherein the forming step comprises heating a mold section associated with the mold cavity (52).

11. The method of any of the preceding claims, wherein the forming step comprises attaching the medical balloon (12) to a catheter shaft (24).

## Patentansprüche

1. Verfahren zur Herstellung eines medizinischen Ballons (12), umfassend:
Bilden des medizinischen Ballons (12) mit einem verlängerten Scoring-Element (60), das aus der Außenoberfläche des Ballons hervorsteht und sich in einer Längsrichtung entlang mindestens eines Gehäuseabschnitts (16) durch Expandieren eines rohrförmigen Vorformlings (50) in einem Formhohlraum (52) erstreckt, der eine Nut (52a, 52b) beinhaltet, die in der Gestalt dem Scoring-Element (60) entspricht, wobei der rohrförmige Vorformling (50) einen konstanten Außendurchmesser aufweist und dem Oberflächenvorsprünge fehlen.

2. Verfahren nach Anspruch 1, wobei der Bildungsschritt das Bilden des Scoring-Elements (60) entlang des Gehäuseabschnitts (16) und mindestens eines Endabschnitts (18, 20) des medizinischen Ballons (12) beinhaltet.

3. Verfahren nach Anspruch 1 oder 2, wobei der Bildungsschritt das Bilden des Scoring-Elements (60) als einzelnes Scoring-Element beinhaltet.

4. Verfahren nach Anspruch 3, wobei der Bildungsschritt das Bilden des Scoring-Elements (60) als einzelnes sich längs erstreckendes Scoring-Element beinhaltet.

5. Verfahren nach Anspruch 1 oder 2, wobei der Bildungsschritt das Bilden einer Vielzahl von Scoring-Elementen (60) beinhaltet.

6. Verfahren nach Anspruch 1, wobei der Bildungsschritt das Bilden des Scoring-Elements (60) als ein sich schraubenförmig erstreckendes Scoring-Element beinhaltet.

7. Verfahren nach einem der vorstehenden Ansprüche, wobei die lineare Nut (52a, 52b) eine umgekehrte V-förmige Kontur zur Bildung des Scoring-Elements mit einem V-förmigen Profil aufweist.

8. Verfahren nach einem der vorstehenden Ansprüche, weiter den Schritt des Bildens der Nut (52a, 52b) in einem Formhohlraum beinhaltend.

9. Verfahren nach einem der vorstehenden Ansprüche, wobei der Bildungsschritt das Anwenden eines unter Druck stehenden Fluids auf ein Inneres des rohrförmigen Vorformlings (50) umfasst.

10. Verfahren nach einem der vorstehenden Ansprüche, wobei der Bildungsschritt das Erhitzen eines Formabschnitts umfasst, der dem Formhohlraum (52) zugeordnet ist.

11. Verfahren nach einem der vorstehenden Ansprüche, wobei der Bildungsschritt das Befestigen des medizinischen Ballons (12) an einem Katheterschaft (24) umfasst.

## Revendications

1. Procédé de fabrication d'un ballonnet médical (12) comprenant :
la formation du ballonnet médical (12) avec un élément de rainurage allongé (60) faisant saillie depuis une surface externe du ballonnet et s'étendant dans une direction longitudinale le long d'au moins une section de cylindre (16) par expansion d'une paraison tubulaire (50) dans une cavité de moule (52) incluant une rainure (52a, 52b) dont la forme correspond à l'élément de rainurage (60), la paraison tubulaire (50) présentant un diamètre externe constant et étant dépourvue de toutes projections de surface.

2. Procédé selon la revendication 1, dans lequel l'étape de formation inclut la formation de l'élément de rainurage (60) le long de la section de cylindre (16) et d'au moins une section d'extrémité (18, 20) du ballonnet médical (12).

3. Procédé selon la revendication 1 ou 2, dans lequel l'étape de formation inclut la formation de l'élément de rainurage (60) sous la forme d'un seul élément de rainurage.

4. Procédé selon la revendication 3, dans lequel l'étape de formation inclut la formation de l'élément de rainurage (60) sous la forme d'un seul élément de rainurage s'étendant longitudinalement.

5. Procédé selon la revendication 1 ou 2, dans lequel l'étape de formation inclut la formation d'une pluralité d'éléments de rainurage (60).

6. Procédé selon la revendication 1, dans lequel l'étape de formation inclut la formation de l'élément de rainurage (60) sous la forme d'un élément de rainurage s'étendant de manière hélicoïdale.

7. Procédé selon l'une quelconque des revendications précédentes, dans lequel la rainure linéaire (52a, 52b) présente un contour en forme de V inversé pour former l'élément de rainurage avec un profil en forme de V.

8. Procédé selon l'une quelconque des revendications précédentes, incluant en outre l'étape de formation de la rainure (52a, 52b) dans une cavité de moule.

9. Procédé selon l'une quelconque des revendications précédentes, dans lequel l'étape de formation comprend l'application d'un fluide sous pression à l'intérieur de la paraison tubulaire (50).

10. Procédé selon l'une quelconque des revendications précédentes, dans lequel l'étape de formation comprend le chauffage d'une section de moule associée à la cavité de moule (52).

11. Procédé selon l'une quelconque des revendications précédentes, dans lequel l'étape de formation comprend la fixation du ballonnet médical (12) à un arbre de cathéter (24).
